# EUROPEAN PATENT APPLICATION

(11) **EP 4 349 375 A1**
(43) Date of publication of application: **10.04.2024**
(21) Application number: 22199915.4
(22) Date of filing: 06.10.2022
(51) Int. Cl.: A61L 2/03

(54) **STERILIZATION AND DISINFECTION DEVICE**

(71) Applicant: Elbiotech Sp. z o.o., 01-919 Warszawa (PL)
(72) Inventor: Pachowicz, Krzysztof, 04-083 Warsaw (PL); Luniewski, Slawomir, 05-830 Kajetany (PL); Baraniak, Joanna, 04-083 Warsaw (PL)
(74) Representative: AOMB Polska Sp. z.o.o.

(57) **Abstract**

The invention belongs to the field of sterilization and disinfection of objects such as drawings, banknotes or medical materials. More specifically, the invention provides a device for ultra-fast and effective disinfection. The device comprises a housing, at least two electrodes, where at least one electrode is a grounded electrode 1 and at least one of the electrodes is a high-voltage electrode 2, a dielectric layer 3 adjoining at least one electrode, a high-voltage supply and a release of electric field impulses. The device is characterized in that at least one electrode adjoins an elastic material 4.

## Description

### Field of the Invention

The invention relates to the field of sterilization and disinfection of objects such as drawings, banknotes, or medical articles. More specifically, the invention provides a device for ultra-fast sterilization and disinfection.

### Prior Art

Devices known from the prior art carry out surface disinfection by means of UV radiation and this does not enable total effective disinfection and sterilization. Other common disinfections concepts are conventional methods - high temperature or alcohol, and this unfortunately leads to permanent damage to some objects being disinfected such as paper or banknotes. This is the kind of materials in which the pathogenic factors remain for the longest time in a form that is dangerous for health. When oxygen-free methods or methods using gases, they are perceived by the users as time consuming methods.

### Subject-matter of the Invention

It is the aim of this invention to provide a device for ultra-fast and effective disinfection of a wide range of objects. Another aim of the invention is to provide better conformity of the device with the irregular shapes of the objects being disinfected.

According to the invention a device for sterilization and disinfection is provided that comprises an outer housing, at least two electrodes, where at least one electrode is a grounded electrode and at least one of the electrodes is a high-voltage electrode, a dielectric layer adjoining at last one of the electrodes, a high-voltage supply, and a release of electric field impulses capable to generate high pulsed voltage on at least one high-voltage electrode, the high-voltage power supply being connected to the impulse release, and the impulse release is connected to at least one high-voltage electrode. The device is characterized in that at least one electrode adjoins an elastic material.

Preferably, the elastic material and all the electrodes are of a cylindrical shape.

In one of the embodiments the elastic material and all the electrodes have a shape of a rectangular prism.

In another embodiment the device comprises three electrodes, one high-voltage electrode and two grounded electrodes.

Preferably, it comprises a vacuum pump to remove air from an operation chamber.

In one of the embodiments, at the at least one electrode there is a heater.

Preferably, the heater is an etched path on a first surface of a PCB printed board, preferably on the other side of the PCB printed board there is a flat electrode.

In another example, the heater has a form of an elastic PCB circuit, preferably the substrate of the elastic PCB circuit being a polyamide substrate.

Preferably, the elastic PCB circuit has a thickness of 0.2 mm.

The dielectric layer is a solid silicone, preferably of a thickness of from 0.5 to 2 mm.

Preferably, the elastic material is a microporous EPDM rubber, preferably of a thickness of 2 to 10 mm.

At least one electrode is made of a copper sheet, preferably of a thickness of from 0.02 to 0.1 mm.

Preferably, at least one electrode is made of an electro conductive fabric, preferably of a thickness of from 0.02 to 0.1 mm.

### Advantageous Effects of the Invention

The object of the invention provided herein enables simple and fast disinfection of objects. The invention is preferably applicable were it is necessary to perform fast disinfection of objects to protect persons against possible infections and spreading of diseases. Preferably, due to magnetic field permeability it is possible to effect total disinfection of an object and it is possible to disinfect fragile objects without damaging them. The electric field used in the subject invention effectively neutralises various kinds of pathogens, i.e. bacteria, viruses or fungi, and at the same time it does not pose any risk for other persons or live organisms in close vicinity.

The provided elastic material allows effective conformity of this material with the object being disinfected. The invention is applicable in various industries, including medicine, banking or archives and museums. Such objects are characterized by shape irregularity. The elastic material will enable complementary conformity with them and effective performing of disinfection.

The device small in size and compact and this makes transporting it easy and enables using it in various places and in other devices. The device is positioned within an electromagnetically sealed housing to protect other devices in the vicinity of the object of the invention. The pulsed electric field used in the subject device does not pose any risk for other persons or live organisms and has a small range that does not exceed the operational area of the device. In order to enhance safety of use, a shielding is provided that may appear outside the operational area.

### Brief Description of the Drawings

The invention will be presented below with reference to the enclosed drawings where:
Fig. 1 - shows the first embodiment of the invention,
Fig. 2 - shows the second embodiment of the invention,
Fig. 3 - shows the third embodiment of the invention.

### Detailed Description of a Preferable Embodiment of the Invention

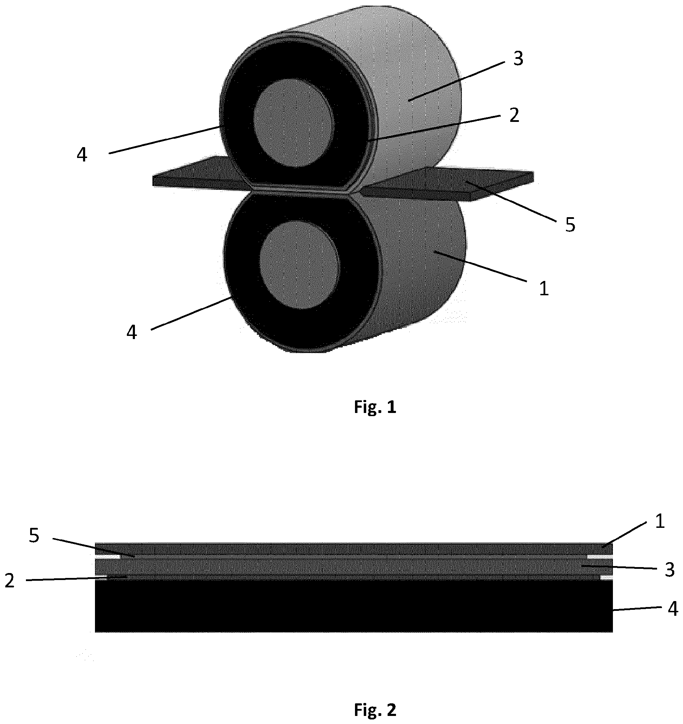

With regard to the enclosed drawings of Figs. 1-3 technical aspects of the device and method for sterilization and disinfection of object will be explained.

The figures show a schematic construction of the device. The device for sterilization and disinfection is built of a housing, electrodes, a dielectric layer 3, a high-voltage supply and a release of electric field impulses. An elastic material 4 adjoins the at least one electrode. The basic factor used for disinfection to deactivate pathogens is a pulsed electric field of a high intensity and suitable parameters.

### Embodiment I

In this embodiment a device for sterilization is applicable in disinfection of banknotes and it is intended to be used, inter alia, in automated teller machines. The device may be used in a method of ultra-fast disinfection of cash in order to decontaminate it before it is supplied to the user.

In this example the device comprises two electrodes - a grounded electrode 1 and a high-voltage electrode 2, both having a cylindrical shape as shown in Fig. 1. The grounded electrode 1 is heated and to the high-voltage electrode 2 a dielectric layer 3 adjoins. At the same time, to the high-voltage electrode 2 a high-voltage supply is connected, and a release of electric field impulses. In this embodiment the dielectric layer 3 is a solid silicone, preferably of a thickness of 0.5-2 mm, which dielectric layer 3 has a cylindrical shape, and the electrodes are made of a copper sheet, preferably of a thickness of 0.02-0.1 mm. In another embodiment, at least one electrode is made of an electroconductive fabric, preferably of a thickness of from 0.02 to 0.1 mm.

In this embodiment, an elastic material 4 adjoins both electrodes - the grounded electrode 1 and the high-voltage electrode 2. Preferably, the elastic material 4 is a microporous rubber EPDM of a thickness of 2-10 mm and it has a cylindrical shape adjusted to the shape of the two electrodes. In operation, i.e. during passing the object 5 being disinfected between the two cylindrical electrodes, the elastic material 4 deforms to adjoin the object as closely as possible.

In this embodiment, at the grounded electrode 1 there is a heater, the heater being in a form of an elastic PCB circuit made on a polyamide substrate. Preferably, the thickness of the PCB circuit is up to 0.2 mm. A person skilled in the art will be aware of other possible embodiments of the heater.

The device, in this embodiment, is applicable in a method for disinfection where high voltage is applied on the high-voltage electrode 2, and this causes generation of an electric field of a high voltage between the two electrodes. The object 5 being disinfected, being a banknote in this embodiment, is passed by rotation of the electrodes and at the same time is being disinfected. Closer positioning of the electrodes relative to each other causes increased pressing against the object 5 being disinfected - a banknote. With the use of a higher pressure force the cylindrical elastic material 4 - in conformity with the shape of the electrode - is locally flattened to form a local flat area of the electrode. In operation of the electrodes, when the two electrodes are pressed against each other, the structure of the elastic layer 4 causes that the electrodes will deform and this will provide a greater surface of effective operation and adjournment to the object 5 being sterilized.

The device of the invention, in this embodiment, may be positioned within an electromagnetically sealed housing to protect other parts of the automated teller machine. In the device a shielding is provided to protect the environment against the effects of the pulsed electric field.

### Embodiment II

In this embodiment, a device for sterilization is applicable in disinfection of drawings or posters and it is intended to be used in museums or archives. The device may be used in a method for ultra-fast disinfection of drawings and posters being porous objects, to eliminate fungi and live organisms.

In this embodiment, the device comprises two electrodes - a grounded electrode 1 and a high-voltage electrode 2, the two electrodes having a shape of a cuboid, as shown in Fig. 2. In this embodiment, the electrodes are copper sheets, preferably of a thickness of from 0.02 to 0.1 mm. An aware user will be aware that other materials may be used for electrodes. In another embodiment, at least one electrode is made of an electroconductive fabric, preferably of a thickness of from 0.02 to 0.1 mm. In this embodiment, the grounded electrode 1 is heated and the high-voltage electrode 2 adjoins a dielectric layer 3 and an elastic material 4. The high-voltage electrode 2 is connected to a high-voltage supply and a release of electric field impulses.

In this embodiment, the elastic material 4 adjoins solely the high-voltage electrode 2 and it is made of a microporous EPDM rubber, preferably of a thickness of 2-10 mm, and the dielectric layer 3, which is positioned between the object 5 being disinfected and the high-voltage electrode 2, is made of solid silicone, preferably of a thickness of 0.5-2 mm. In this embodiment, the elastic material 4 and the dielectric layer 3 have a shape of a cuboid. The shape of the elastic material 4 corresponds to the shape of the electrode it adjoins.

The grounded electrode 2 is additionally heated and, in this embodiment, a heater is used that has a form of an etched path on a first surface of a PCB printed circuit, and a person skilled in the art will be aware that also other embodiments of the heater are possible.

The device, in this embodiment, is applicable in a method for disinfection where high voltage is applied on the high-voltage electrode 2 and this generates high voltage electric field between the two electrodes with the object 5 being disinfected therebetween.

In this embodiment, the electrodes closely adjoin the object 5 being disinfected, by means of the elastic material 4 they are made from and due to the properties of the elastic material 4 adjoining the high-voltage electrode. The elastic material 4 along with the electrode deforms in operation, to adjoin possibly closely the object 5 being disinfected to provide the best effectiveness of disinfection and sterilization. In order to enhance adjoining of the electrodes, in this example, a vacuum pump is used to remove air from the space between the electrodes - from the operation chamber, and the pressure therein is decreased or increased (as needed). In another embodiment, the air may be replaced with another gas having microbicide properties or having better dielectric properties than the air. The use of the vacuum pump enables pumping in or out from the operational chamber between the electrodes to enable better adjoining the object being disinfected.

The device provided by the invention, in this example, may be placed in an electromagnetically sealed housing to protect other devices positioned nearby. In the device a shielding is used to shield the environment against the effects of the pulsed electric field.

### Embodiment III

In this embodiment, a device for sterilization is applicable in disinfection of porous medical materials such as surgical thread or surgical cloth, and is applicable in production lines.

In this example, the device comprises at least two electrodes - a grounded electrode 1 and a high-voltage electrode 2. The shape of the electrodes is adjusted to the objects 5 being sterilized. The electrodes can have a shape of a cuboid - if the object 5 subject to disinfection is e.g. a surgical cloth. a cylinder - of the object subject to disinfection is e.g. a thread. In other embodiments, electrodes may have other, more diversified shapes, that are adjusted to the object to enable the most effective disinfection. In this embodiments, electrodes are made of a copper sheet, preferably of a thickness of 0.01-0.1 mm. In another embodiment, at least one electrode is made of an electrocoductive fabric, preferably of a thickness of from 0.02 to 0.1 mm.

One of the electrodes, in this embodiment the grounded electrode 1, is heated. Heating is effected by means of a heater which is in a form of an etched path on a first surface of a PCB printed circuit PCB in the case in which the electrode has a shape of a cuboid. In another embodiment, when the electrodes have more diversified shapes, the heater is in a form or an elastic PCB circuit, preferably made on a polyamide substrate and of a thickness of up to 0.2 mm, and a person skilled in the art will be aware that also other embodiments of the heater are possible.

The second electrode is a high-voltage electrode 2 that is connected to a high-voltage supply and to a release of electric field impulses.

In this embodiment, to the both kinds of electrodes - the grounded electrode 1 and the high-voltage electrode 2, an elastic material 4 adjoins. Preferably, the elastic material 4 is a microporous EPDM rubber of a thickness of 2-10 mm and it has a shape adjusted in conformity with the shape of the electrodes. In the case in which the electrodes have a cylindrical shape, the elastic material 4 has such a shape and the object being disinfected is passed between the two cylindrical electrodes. The grounded electrode 1 is heated and to the high-voltage electrode 2 a dielectric layer 3 adjoins. In this example, the dielectric layer 3 is a solid silicone, preferably of a thickness of 0.5-2 mm, which dielectric layer 3 has a cylindrical shape.

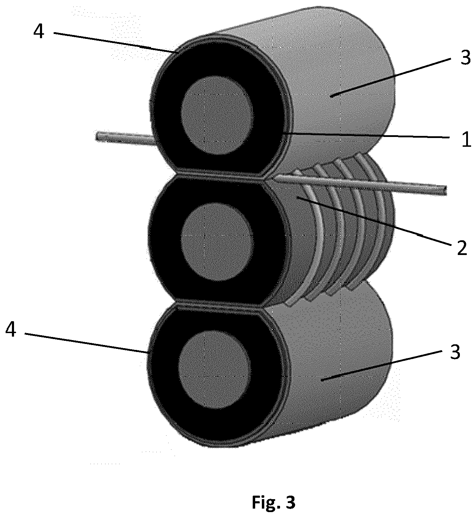

A further embodiment, shown in Fig. 3, uses two grounded electrodes 1 between which there is one high-voltage electrode 2 and all they of a cylindrical shape. The elastic material 4 which adjoins each of the electrodes is adjusted according to the shape of the electrodes and it has a cylindrical shape. The dielectric layer adjoins the grounded electrode 1 and the material subject to disinfection, in this case being a surgical thread, wraps the electrode and adjoins the dielectric layer 3 as shown in Fig. 3.

The device, in this embodiment is applicable in a method for disinfection in which high voltage is applied on the high-voltage electrode 2 causing generation of a high voltage electric field between the two electrodes.

In order to enhance adjoining of the electrodes, in this example a vacuum pump is used to remove air from the space between the electrodes - from the operational chamber, and decreases or increases the pressure therein. In another example the air mat be replaced with another gas of microbicide properties or better dielectric properties than the air. The use of a vacuum pump enables pumping the air in or out from the operational chamber between the electrodes and this enables better adjoining the object being disinfected.

The device provided by the invention, in this example, may be placed in an electromagnetically sealed housing to protect other devices positioned nearby. In the device a shielding is used to shield the environment against the effects of the pulsed electric field.

## Claims

1. A device for sterilization and disinfection comprising:
a housing,
at least two electrodes, where at least one electrode is a grounded electrode (1) and at least one of the electrodes is a high-voltage electrode (2),
a dielectric layer (3) adjoining at least one electrode,
a high-voltage supply and a release of electric field impulses, which are capable to generate high pulsed voltage on at least one high-voltage electrode (2),
and the high-voltage supply is connected to the impulse release, and the impulse release is connected to at least one high-voltage electrode (2),
**characterized in that** at least one electrode adjoins the elastic material (4).

2. Device according to claim 1 **characterized in that** the elastic material (4) and all the electrodes have a cylindrical shape.

3. Device according to claims 1 or 2 **characterized in that** the elastic material (4) and all the electrodes have a shape of a cuboid.

4. Device according to claims 1 or 2 **characterized in that** the device comprises three electrodes, one high-voltage electrode (2) and two grounded electrodes (1).

5. Device according to claims 1 to 4 **characterized in that** it comprises a vacuum pump to remove air from the operational chamber.

6. Device according to any one of claims from 1 to 5 **characterized in that** at the at least one electrode there is a heater.

7. Device according to any one of claims from 1 to 6 **characterized in that** the heater is an etched path on a first surface of a PCB printed board, preferably on the other surface of the PCB printed board a flat electrode is arranged.

8. Device according to any one of claims from 1 to 7 **characterized in that** the heater has a form of an elastic PCB circuit, preferably the substrate of the elastic PCB circuit being a polyamide substrate.

9. Device according to claim 7 **characterized in that** the elastic PCB circuit has a thickness of up to 0.2 mm.

10. Device according to any one of claims 1-9 **characterized in that** the dielectric layer (3) is a solid silicone, preferably of a thickness of from 0.5 to 2 mm.

11. Device according to any one of claims from 1-10 **characterized in that** the elastic layer (4) is a microporous rubber EPDM, preferably of a thickness of 2 to 10 mm.

12. Device according to any one of claims from 1-11 **characterized in that** at least one electrode is made of a copper sheet, preferably of a thickness of from 0.02 to 0.1 mm.

13. Device according to any one of claims from 1-11 **characterized in that** at least one electrode is made of an electroconductive fabric, preferably of a thickness of from 0.02 to 0.1 mm.
